# FASCICULE DE BREVET EUROPEEN

(11) **EP 3 003 405 B1**
(45) Date de publication et mention de la délivrance du brevet: **19.05.2021**
(21) Numéro de dépôt: 14727504.4
(22) Date de dépôt: 30.05.2014
(51) Int. Cl.: A61L 2/08

(54) **INSTALLATION DE TRAITEMENT D'ARTICLES PAR BOMBARDEMENT ÉLECTRONIQUE**
VORRICHTUNG ZUR BEHANDLUNG VON GEGENSTÄNDEN MITTELS ELEKTRONISCHEN BESCHUSSES
EQUIPMENT FOR TREATING ITEMS BY MEANS OF ELECTRONIC BOMBARDMENT

(30) Priorité: 03.06.2013 FR 1355040
(43) Date de publication de la demande: 13.04.2016
(73) Titulaire: Serac Group, 72400 La Ferté-Bernard (FR)
(72) Inventeur: GESLOT, Nicolas, 72540 Saint-Christophe-en-Champagne (FR)
(74) Mandataire: Lavialle, Bruno François Stéphane
(86) Numéro de dépôt international: PCT/EP2014/061294
(87) Numéro de publication internationale: WO 2014/195240

(56) Documents cités:
- EP-A1- 2 371 397
- WO-A1-2013/058205
- WO-A1-2013/068251
- US-A1- 2011 012 030
- US-B1- 6 529 577

## Description

La présente invention concerne une installation de traitement d'articles par rayonnement et plus particulièrement par bombardement électronique.

Il est connu de stériliser des récipients en soumettant ceux-ci à un bombardement électronique (EP2371397).

Ce bombardement électronique est réalisé dans une enceinte blindée visant à confiner les rayons X dans une zone dont sont exclus les opérateurs.

A cette fin, une installation de traitement de récipients par bombardement électronique, comprend un bâti sur lequel est monté un dispositif de transport de récipients dont une partie est logée dans une enceinte blindée au voisinage d'un ou plusieurs émetteurs de rayonnement. L'enceinte comporte une entrée et une sortie formant passage pour le dispositif de transport.

Pour affaiblir l'énergie des rayons X émis par l'émetteur et les empêcher de sortir de l'enceinte blindée, il est connu de disposer dans l'enceinte des cloisons internes blindées agencées en chicane horizontale vers l'entrée et la sortie de l'enceinte blindée. Par un agencement en chicane, on entend que les cloisons internes sont disposées pour interdire un trajet direct des rayons X de l'émetteur jusqu'à l'entrée et jusqu'à la sortie. Ainsi, les rayons X produits dans l'environnement de l'émetteur rebondissent contre les parois de l'enceinte blindée et les cloisons internes blindées un nombre suffisant de fois pour avoir perdu l'essentiel de leur énergie avant d'avoir atteint l'entrée et la sortie de l'enceinte blindée.

La mise en place de cloisons internes oblige à utiliser des dispositifs intermédiaires de transfert de récipients pour permettre le passage des chicanes.

Si la protection des opérateurs est très bien assurée dans ces installations, la présence des chicanes est très contraignante. Les installations présentent l'inconvénient d'être très encombrantes au sol notamment en raison de la présence des dispositifs intermédiaires de transfert dont les dimensions influent directement sur l'encombrement au sol de l'installation.

Un but de l'invention est de fournir un moyen pour protéger les opérateurs intervenant au voisinage d'installation de traitement d'articles par rayonnement tout en limitant l'encombrement de l'installation.

A cet effet, on prévoit, selon l'invention, une installation de traitement d'articles par rayonnement, comme décrit dans les revendications.

Ainsi, l'agencement vertical de la chicane permet d'assurer une étanchéité de l'enceinte en limitant l'encombrement au sol de l'installation. On notera que les articles entrent et sortent de l'enceinte en étant conduits par le dispositif de transport. En application à des récipients, ceci facilite l'implantation de la station de traitement dans une installation de conditionnement autorisant une cadence relativement élevée (par opposition à une installation de traitement de laquelle les récipients seraient évacués en vrac, ce qui facilite l'agencement des blindages mais limite la cadence de production dans le cas d'une implantation dans une installation de conditionnement).

De préférence, le passage en chicane est défini par des parois s'étendant transversalement en saillie dans le tunnel pour définir des ouvertures de passage successivement décalées en hauteur.

On obtient alors par exemple une ouverture supérieure entre deux ouvertures inférieures ou inversement. Ce mode de réalisation facilite l'implantation du dispositif de transport dans le tunnel tout en limitant la longueur du tunnel.

L'invention a également pour objet une installation de conditionnement d'un produit dans des récipients, comportant une telle installation de traitement reliée à une station amont et à une station aval par les tronçons du dispositif de transport s'étendant dans ledit au moins un tunnel.

La station amont est par exemple une station de nettoyage des récipients et la station aval est par exemple une station de remplissage des récipients.

D'autres caractéristiques et avantages de l'invention ressortiront à la lecture de la description qui suit de modes de réalisation particuliers non limitatifs de l'invention.

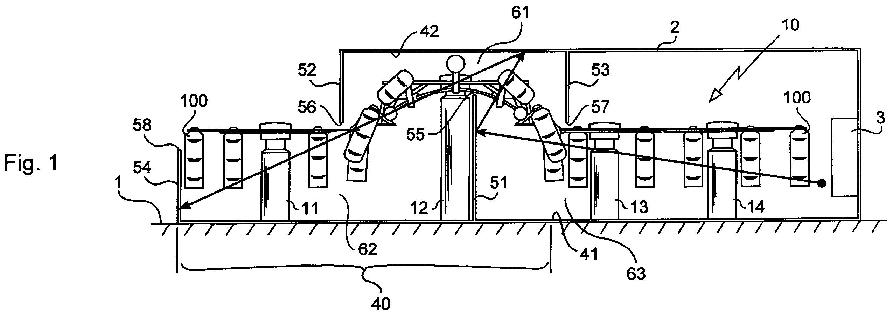

Il sera fait référence à la figure unique annexée représentant schématiquement une partie d'une installation conforme à l'invention.

En référence à la figure, l'installation de traitement conforme à l'invention est ici agencée pour la stérilisation de récipients par soumission de ceux-ci à un bombardement électronique.

L'installation de l'invention est destinée à prendre place dans une ligne de production, par exemple en aval d'une station de soufflage des récipients et en amont d'une station de remplissage desdits récipients.

L'installation comprend un bâti 1 sur lequel est monté un dispositif, généralement désigné en 10, de transport de récipients 100.

Une partie du dispositif de transport 1 est logée dans une enceinte blindée 2 au voisinage d'un émetteur de rayonnement 3 contenu dans l'enceinte 2. L'émetteur de rayonnement 3 assure ici un bombardement électronique.

L'enceinte comporte un tunnel d'accès 40 qui comprend des cloisons internes blindées qui s'étendent transversalement au tunnel 40 pour délimiter un passage en chicane, généralement désigné en 50, dans lequel s'étend un tronçon du dispositif de transport 10.

Plus précisément, une première 51 des cloisons s'étend depuis une surface inférieure 41 du tunnel 40 vers une surface supérieure 42 du tunnel 40 entre une deuxième cloison 52 et une troisième cloison 53 s'étendant toutes deux depuis la surface supérieure 42 vers la surface inférieure 41. Les cloisons 51, 52, 53 sont sensiblement parallèles les unes aux autres. La première cloison 51 a un bord supérieur 55 qui délimite une première ouverture 61 avec la surface supérieure 42. La deuxième cloison 52 et la troisième cloison 53 ont un bord inférieur 56, 57 délimitant avec la surface inférieure 41 respectivement une deuxième ouverture 62 et une troisième ouverture 63 avec la surface inférieure 41. Le bord supérieur 55 de la première cloison 51 s'étend au-dessus du bord inférieur 56, 57 de la deuxième cloison 52 et de la troisième cloison 53. Les cloisons 51, 52, 53 sont écartées des unes des autres et sont ainsi disposées pour interdire un trajet direct des rayons X de l'émetteur de rayonnement 3 jusqu'à l'extérieur de l'enceinte 2. Ledit dispositif de transport 10 possède des moyens permettant le passage de la chicane par les-dits articles par dessus le bord supérieur 55 de la cloison 51.

Le tunnel comprend ici une quatrième cloison 54 blindée qui s'étend transversalement au tunnel 40 à l'extrémité de celui-ci située le plus à l'extérieur de l'enceinte 2. La quatrième cloison 54 s'étend depuis la surface inférieure 41 et a son bord supérieur 58 s'étendant en dessous du bord inférieur 56 de la deuxième cloison 52. La quatrième cloison 54 vient renforcer l'étanchéité de l'enceinte 2 et délimite avec la deuxième cloison 52 une ouverture d'entrée/sortie du tunnel 40.

Par blindée, on entend que la paroi ou cloison est étanche aux électrons et rayons X. La paroi ou cloison blindée comprend ainsi une couche de plomb recouverte d'une feuille d'acier inoxydable.

Le dispositif de transport 10 comprend ici des étoiles de transport 11, 12, 13, 14, connues en elles-mêmes, montées pour tourner sur le bâti 1 et reliées à un dispositif de leur entraînement en rotation non représenté. Les étoiles de transport 11, 12, 13, 14 sont tangentes les unes aux autres et sont pourvues chacune de moyens de préhension des récipients, tels que des pinces de saisie des récipients par le col.

L'étoile de transport 11 est située entre la quatrième cloison 54 et la deuxième cloison 52. Elle participe à l'introduction des récipients 100 dans le tunnel 40 ou l'évacuation des récipients 100 hors du tunnel 40. L'étoile de transport 11 est tangente à au moins un dispositif de transport de récipients, non représenté, agencé pour charger et décharger l'étoile de transport 11.

L'étoile de transport 12 est montée au voisinage de la première cloison 51 et pourvue de moyens pour faire basculer les récipients par-dessus le bord supérieur 55 de la première cloison 51. L'étoile comprend une plateforme s'étendant au-dessus du bord supérieur 55 à cheval sur celui-ci entre les deuxième et troisième cloisons 52, 53. Les pinces de saisie des récipients sont montées sur la plateforme de l'étoile de transport 12 pour pivoter autour d'un axe horizontal entre une position de maintien des récipients en position verticale et une position de maintien des récipients en position horizontale de telle manière que les récipients puissent être passés par-dessus le bord supérieur 55 en position sensiblement horizontale et pour échanger les récipients en position verticale avec l'étoile de transport 11 et l'étoile de transport 13 située au voisinage de la troisième cloison 53.

Les étoiles de transport 13 et 14 ont leur axe de rotation du côté de la troisième cloison 53 opposé à la deuxième cloison 52. La plateforme de l'étoile de transport 13 se trouve à cheval sous le bord inférieur 57. L'étoile de transport 14 est adjacente à l'émetteur de rayonnement 3.

Les plateformes des étoiles de transport 11, 13, 14 sont au même niveau. Les pinces de saisie, de l'étoile de transport 12, sont également au même niveau que lesdites plateformes lorsque lesdites pinces sont dans leur position de maintien des récipients en position verticale.

De telles étoiles sont connues en elles-mêmes et ne seront donc pas plus détaillées ici.

Bien entendu, l'invention n'est pas limitée aux modes de réalisation décrits mais englobe toute variante entrant dans le champ de l'invention telle que définie par les revendications.

En particulier, le dispositif de transport peut comprendre des étoiles de transport, des convoyeurs linéaires ou tout autre appareil permettant de transporter les récipients.

L'installation peut comprendre un nombre différent d'étoiles.

L'agencement des cloisons peut être différent de celui décrit. Plus précisément, la première des cloisons s'étend depuis la surface supérieure 42 du tunnel 40 vers la surface inférieure 41 du tunnel 40 entre la deuxième cloison et la troisième cloison s'étendant toutes deux depuis la surface inférieure 41 vers la surface supérieure 42. La première cloison a alors un bord inférieur qui délimite la première ouverture avec la surface inférieure 41. La deuxième cloison et la troisième cloison ont un bord supérieur délimitant avec la surface supérieure respectivement la deuxième ouverture et la troisième ouverture avec la surface supérieure 42. Le bord inférieur de la première cloison s'étend en-dessous du bord supérieur de la deuxième cloison et de la troisième cloison. Le tunnel pourrait être également défini par des parois supérieure et inférieure s'étendant en zig-zag pour délimiter le passage en chicane vertical. Cependant, cet agencement est plus complexe à fabriquer que ceux précédemment décrits.

L'installation peut comprendre un tunnel d'entrée et un tunnel de sortie, ou un tunnel unique formant à la fois l'entrée et la sortie de l'installation.

L'installation peut comprendre des émetteurs embarqués sur l'étoile 14 et/ou un ou plusieurs émetteurs fixes, uniquement un ou plusieurs émetteurs fixes, ou uniquement des émetteurs embarqués sur l'étoile 14.

Les étoiles peuvent avoir une structure différente de celle décrite notamment en ce qui concerne la préhension des récipients.

L'invention peut être appliquée à d'autres articles que des récipients et/ou à d'autres types de rayonnement.

## Revendications

1. Installation de traitement d'articles par rayonnement, comprenant :
(a) un bâti (1)
(b) une enceinte blindée (2) qui comporte:
(b1) au moins un tunnel (40),
(b2) au moins un émetteur de rayonnement (3),
(c) un dispositif de transport (10) d'articles monté dans ledit tunnel (40), et dont une partie est logée dans ladite enceinte blindée (2) au voisinage dudit au moins émetteur de rayonnement (3),
**caractérisée en ce que** ledit tunnel comporte un passage en chicane (50) pour assurer l'étanchéité de l'enceinte en rayonnement, ladite chicane étant définie par:
- une cloison intermédiaire (51) qui s'étend depuis une surface inférieure (41) du tunnel (40) vers une surface supérieure (42) du tunnel (40), ladite cloison intermédiaire (51) ayant un bord supérieur (55) qui délimite une première ouverture (61) avec la surface supérieure (42),
- deux cloisons (52, 53) qui encadrent ladite cloison intermédiaire et qui s'étendent depuis la surface supérieure (42) du tunnel (40) vers la surface inférieure (41) du tunnel (40), et ayant chacune un bord inférieur (56, 57) délimitant avec la surface inférieure (41) respectivement une deuxième ouverture (62) et une troisième ouverture (63) avec la surface inférieure (41), le bord supérieur (55) de la cloison intermédiaire (51) s'étendant au-dessus du bord inférieur (56, 57) des deux cloisons (52, 53) qui l'entourent, ledit dispositif de transport (10) possède des moyens permettant le passage de la chicane par les-dits articles par dessus le bord supérieur (55) de la cloison intermédiaire (51).

2. Installation selon la revendication 1 dans laquelle le dispositif de transport comprend une étoile rotative montée au voisinage de la cloison intermédiaire et pourvue de moyens pour faire basculer les récipients par-dessus le bord supérieur de la cloison intermédiaire.

3. Installation de traitement d'articles par rayonnement, comprenant :
(a) un bâti (1)
(b) une enceinte blindée (2) qui comporte:
(b1) au moins un tunnel (40),
(b2) au moins un émetteur de rayonnement (3),
(c) un dispositif de transport (10) d'articles monté dans ledit tunnel (40), et dont une partie est logée dans ladite enceinte blindée (2) au voisinage dudit au moins émetteur de rayonnement (3),
**caractérisée en ce que** ledit tunnel comporte un passage en chicane (50) pour assurer l'étanchéité de l'enceinte en rayonnement, ladite chicane étant définie par:
- une cloison intermédiaire (51) qui s'étend depuis une surface supérieure (41) du tunnel (40) vers une surface inférieure (42) du tunnel (40), ladite cloison intermédiaire (51) ayant un bord inférieur qui délimite une première ouverture avec la surface inférieure (42),
- deux cloisons (52, 53) qui encadrent ladite cloison intermédiaire et qui s'étendent depuis la surface inférieure (42) du tunnel (40) vers la surface supérieure (41) du tunnel (40), et ayant chacune un bord supérieur délimitant avec la surface supérieure (42) respectivement une deuxième ouverture et une troisième ouverture avec la surface supérieure (42), le bord inférieur de la cloison intermédiaire (51) s'étendant en-dessous du bord supérieur des deux cloisons (52, 53) qui l'entourent, ledit dispositif de transport (10) possède des moyens permettant le passage de la chicane par les-dits articles par dessous le bord inférieur de la cloison intermédiaire (51).

4. Installation selon la revendication 3, dans laquelle le dispositif de transport comprend une étoile rotative montée au voisinage de la première cloison et pourvue de moyens pour faire basculer les récipients par-dessous le bord inférieur de la cloison intermédiaire.

5. Installation selon l'une des revendications précédentes, dans laquelle le passage en chicane est défini par des parois s'étendant transversalement en saillie dans le tunnel pour définir des ouvertures de passage successivement décalées en hauteur.

6. Installation selon l'une des revendications précédentes,
dans laquelle le tunnel (40) comprend une quatrième cloison (54) blindée qui s'étend transversalement au tunnel (40) et est située à l'extrémité de celui-ci et le plus à l'extérieur de l'enceinte (2). La quatrième cloison (54) s'étend depuis la surface inférieure (41) et a son bord supérieur (58) s'étendant en dessous du bord inférieur (56) de la deuxième cloison (52).

7. Installation de conditionnement d'un produit dans des récipients, comportant une installation de traitement selon l'une quelconque des revendications précédentes, l'installation de traitement étant reliée à une station amont et à une station aval par les tronçons du dispositif de transport s'étendant dans ledit au moins un tunnel.

## Patentansprüche

1. Anlage zum Behandeln von Artikeln mittels Strahlung, umfassend:
(a) ein Gestell (1)
(b) eine abgeschirmte Einfassung (2), die umfasst:
(b1) mindestens einen Tunnel (40),
(b2) mindestens einen Strahlungssender (3),
(c) eine Transportvorrichtung (10) zum Transport von Artikeln, die in dem genannten Tunnel (40) montiert ist und von der ein Teil in der genannten abgeschirmten Einfassung (2) in der Nähe des genannten mindestens einen Strahlungssenders (3) untergebracht ist,
**dadurch gekennzeichnet, dass** der genannte Tunnel einen Schrankendurchlass (50) umfasst, um die Strahlungsdichtheit der Einfassung sicherzustellen, wobei die Schranke definiert ist durch:
- eine Zwischentrennwand (51), die sich von einer unteren Fläche (41) des Tunnels (40) in Richtung einer oberen Fläche (42) des Tunnels (40) erstreckt, wobei die genannte Zwischentrennwand (51) einen oberen Rand (55) hat, der mit der oberen Fläche (42) eine erste Öffnung (61) begrenzt,
- zwei Trennwände (52, 53), die die Zwischentrennwand einrahmen und sich von der oberen Fläche (42) des Tunnels (40) in Richtung der unteren Fläche (41) des Tunnels (40) erstrecken und jeweils einen unteren Rand (56, 57) haben, der mit der unteren Fläche (41) eine zweite Öffnung (62) bzw. mit der unteren Fläche (41) eine dritte Öffnung (63) begrenzt, wobei der obere Rand (55) der Zwischentrennwand oberhalb des unteren Rands (56, 57) der beiden Trennwände (523, 53) verläuft, die sie umschließen, wobei die Transportvorrichtung (10) Mittel hat, die den Durchtritt der genannten Artikel durch den Schrankendurchlass über den oberen Rand (55) der Zwischentrennwand (51) hinweg gestatten.

2. Anlage nach Anspruch 1, bei der die Transportvorrichtung einen Drehstern umfasst, der in der Nähe der Zwischentrennwand montiert und mit Mitteln versehen ist, um die Behälter über den oberen Rand der Zwischentrennwand hinweg zu kippen.

3. Anlage zum Behandeln von Artikeln mittels Strahlung, umfassend:
(a) ein Gestell (1)
(b) eine abgeschirmte Einfassung (2), die umfasst:
(b1) mindestens einen Tunnel (40),
(b2) mindestens einen Strahlungssender (3),
(c) eine Transportvorrichtung (10) zum Transport von Artikeln, die in dem genannten Tunnel (40) montiert ist und von der ein Teil in der genannten abgeschirmten Einfassung (2) in der Nähe des genannten mindestens einen Strahlungssenders (3) untergebracht ist,
**dadurch gekennzeichnet, dass** der genannte Tunnel einen Schrankendurchlass (50) umfasst, um die Strahlungsdichtheit der Einfassung sicherzustellen, wobei die genannte Schranke definiert ist durch:
- eine Zwischentrennwand (51), die sich von einer oberen Fläche (41) des Tunnels (40) in Richtung einer unteren Fläche (42) des Tunnels (40) erstreckt, wobei die genannte Zwischentrennwand (51) einen unteren Rand hat, der mit der unteren Fläche (42) eine erste Öffnung begrenzt,
- zwei Zwischentrennwände (52, 53), die die genannte Zwischentrennwand einrahmen und sich von der unteren Fläche (42) des Tunnels (40) in Richtung der oberen Fläche (41) des Tunnels (40) erstrecken und jeweils einen oberen Rand haben, der mit der oberen Fläche (42) eine zweite Öffnung bzw. mit der oberen Fläche (42) eine dritte Öffnung begrenzt, wobei der untere Rand der Zwischentrennwand (51) unterhalb des oberen Rands der beiden Trennwände (52, 53) verläuft, die sie umgeben, wobei die genannte Transportvorrichtung (10) Mittel besitzt, die den Durchtritt der genannten Artikel durch den Schrankendurchlass unter dem unteren Rand der Zwischentrennwand (51) hindurch gestatten.

4. Anlage nach Anspruch 3, bei der die Transportvorrichtung einen Drehstern umfasst, der in der Nähe der ersten Zwischenwand montiert und mit Mitteln versehen ist, um die Behälter unter dem unteren Rand der Zwischenwand hindurch zu kippen.

5. Anlage nach einem der vorhergehenden Ansprüche, bei der der Schrankendurchlass von Wänden definiert ist, die quer in den Tunnel vorstehen, um Durchlassöffnungen zu definieren, die in der Höhe sukzessive versetzt sind.

6. Anlage nach einem der vorhergehenden Ansprüche, bei der der Tunnel (40) eine vierte abgeschirmte Trennwand (54) umfasst, die quer zum Tunnel (40) verläuft und sich am Ende desselben befindet und am weitesten außerhalb der Einfassung (2), wobei sich die vierte Trennwand (54) von der unteren Fläche (41) erstreckt und sich ihr oberer Rand (58) unterhalb des unteren Rands (56) der zweiten Trennwand (52) erstreckt.

7. Anlage zum Abpacken eines Produktes in Behälter, umfassend eine Behandlungsanlage nach einem der vorhergehenden Ansprüche, wobei die Behandlungsanlage über die Abschnitte der Transportvorrichtung, die sich in dem genannten mindestens einen Tunnel erstrecken, mit einer stromaufwärtigen Station und einer stromabwärtigen Station verbunden ist.

## Claims

1. Installation for treating articles by radiation, comprising:
(a) a structure (1),
(b) a shielded enclosure (2) comprising:
(b1) at least one tunnel (40),
(b2) at least one radiation emitter (3),
(c) an article transport device (10) mounted in said tunnel (40), with a portion housed in said shielded enclosure (2) in the vicinity of at least one radiation emitter (3),
**characterized in that** said tunnel (40) comprises a baffle passage (50) for ensuring that the enclosure is radiation-proof, said baffle passage being defined by:
- an intermediate partition (51) extending from a bottom surface (41) of the tunnel (40) towards a top surface (42) of the tunnel (40), the intermediate partition having a top edge (55) defining a first opening (61) with the top surface,
- two partitions (52, 53) framing the intermediate partition (51) and extending from the top surface (42) of the tunnel (40) towards the bottom surface (41) and having bottom edges (56, 57) defining respective second and third openings (62, 63) with the bottom surface, the top edge (55) of the intermediate partition (51) being higher than the bottom edges (56, 57) of the second and third partitions (52, 53) that frame the intermediate partition (51), said transport device (10) comprising means for making the articles passing the baffle passage above the top edge (55) of the intermediate partition (51).

2. Installation according to claim 1, wherein the transport device (10) comprises a rotary star mounted in the vicinity of the intermediate partition and provided with means for causing the articles to tilt over the top edge of the intermediate partition.

3. Installation for treating articles by radiation, comprising:
(a) a structure (1),
(b) a shielded enclosure (2) comprising:
(b1) at least one tunnel (40),
(b2) at least one radiation emitter (3),
(c) an article transport device (10) mounted in said tunnel (40), with a portion housed in said shielded enclosure (2) in the vicinity of at least one radiation emitter (3),
**characterized in that** said tunnel (40) comprises a baffle passage (50) for ensuring that the enclosure is radiation-proof, said baffle passage being defined by:
- an intermediate partition (51) extending from a top surface (42) of the tunnel (40) towards a bottom surface (41) of the tunnel (40), the intermediate partition having a bottom edge defining a first opening with the bottom surface,
- two partitions (52, 53) framing the intermediate partition (51) and extending from the bottom surface (42) of the tunnel(40) towards the top surface (42) and having top edges defining respective second and third openings with the top surface (42), the bottom edge of the intermediate partition (51) being lower than the top edges (56, 57) of the second and third partitions (52, 53) that frame the intermediate partition (51), said transport device (10) comprising means for making the articles passing the baffle passage under the bottom edge of the intermediate partition (51).

4. Installation according to claim 3, wherein the transport device (10) comprises a rotary star is mounted in the vicinity of the first partition and provided with means for causing the articles to tilt under the bottom edge of the intermediate partition.

5. Installation according to anyone of the preceding claims, wherein the baffle passage is defined by partitions transversely protruding in the tunnel for defining openings successively offset vertically.

6. Installation according to anyone of the preceding claims, wherein the tunnel (40) has a shielded fourth partition (54) extending transversely relative to the tunnel (40) at its end that is situated furthest out from the enclosure (2), the fourth partition (54) extending from the bottom surface (41) and having its top edge (58) extending below the bottom edge (56) of the second partition (52).

7. Packaging installation for packaging a substance in containers, including a treatment installation according to anyone of the preceding claims, the treatment installation being connected to an upstream station and to a downstream station by segments of the transport device extending in said at least one tunnel.
